# EUROPEAN PATENT SPECIFICATION

(11) **EP 3 009 447 B1**
(45) Date of publication and mention of the grant of the patent: **06.06.2018**
(21) Application number: 15191348.0
(22) Date of filing: 23.07.2010
(51) Int. Cl.: C07K 14/505, C12P 21/00, C12N 5/00

(54) **PRODUCTION OF ERYTHROPOIESIS STIMULATING PROTEIN USING METAL IONS**
HERSTELLUNG VON ERYTHROPOESESTIMULIERENDEM PROTEIN UNTER VERWENDUNG VON METALLIONEN
PRODUCTION DE PROTÉINE DE STIMULATION DE L'ÉRYTHROPOÏÈSE AU MOYEN D'IONS MÉTALLIQUES

(30) Priority: 24.07.2009 IN 174109
(43) Date of publication of application: 20.04.2016
(62) Divisional of application: 10802943.0
(73) Proprietor: Dr. Reddy's Laboratories, Ltd., Hyderabad 500 016 AP (IN); Dr. Reddy's Laboratories Inc., Bridgewater, NJ 08807 (US)
(72) Inventor: PANKHANIA, Ashvin, 380 058 Gujurat (IN); REDDY, Degalahal, Ganesh, 500 072 Andhra Pradesh (IN); KAMARAJU, Mamata, 500 079 Andhra Pradesh (IN)
(74) Representative: Mummery, Thomas Zack

(56) References cited:
- WO-A2-03/045995
- US-A1- 2007 161 084
- American Type Culture Collection: "Formulation for Dulbecco's Modified Eagle's Medium (DMEM) ATCC® 30-2002", , XP002754739, Retrieved from the Internet: URL:http://www.lgcstandards-atcc.org/~/med ia/D7399222FD8B4EF68FD56DD8058804BA.ashx [retrieved on 2016-02-25]
- MARTIN GAWLITZEK ET AL: "Identification of cell culture conditions to control N-glycosylation site-occupancy of recombinant glycoproteins expressed in CHO cells", BIOTECHNOLOGY AND BIOENGINEERING, WILEY & SONS, HOBOKEN, NJ, US , vol. 103, no. 6 9 April 2009 (2009-04-09), pages 1164-1175, XP002688571, ISSN: 0006-3592, DOI: 10.1002/BIT.22348 Retrieved from the Internet: URL:http://onlinelibrary.wiley.com/doi/10. 1002/bit.22348/pdf [retrieved on 2009-04-07]

## Description

### INTRODUCTION

This invention relates to culture methods for the production of erythropoiesis stimulating proteins.

Erythropoiesis stimulating proteins, such as erythropoietin and analogs of erythropoietin are glycoprotein hormones that are the principle homeostatic regulators of red blood cell production. Natural erythropoietin, or EPO, is produced by the kidney; but its high demand for therapeutic purposes has led to large scale production by recombinant DNA methods. Purified recombinant human EPO is administered to human patients for the treatment of medical indications associated with inadequate red blood cell supply, such as anemia and chronic renal failure. Recombinant human erythropoietin (rHuEPO) and its analog darbepoetin alfa are used clinically to treat anemia and increase red blood cell production in various conditions, such as perisurgery, chronic renal failure, side effects of HIV or HCV treatment, and side effects of cancer chemotherapy.

The recombinant expression and production of glycoproteins, such as EPO and analogs of EPO, is complicated by the need for both high level expression, as well as appropriate post translation processing of these glycoproteins. An important post-translational processing that occurs subsequent to cellular protein expression is glycosylation, wherein glycans are enzymatically attached to the polypeptide backbone. Protein glycosylation is of two general types: O-linked glycosylation, wherein the glycan group is attached to the hydroxyl oxygen of serine or threonine side chains; and N-linked glycosylation, wherein the glycan group is attached to amide nitrogen of aspargine side chains. The nature and composition of the sugars that makeup the glycan group can significantly affect the physico-chemical properties, isoform composition and biological activities of glycoproteins. For instance N-acetylneuraminic acid (hereinafter referred to as sialic acid) is one of the most commonly present sugars in the glycan moiety of glycoproteins. Being negatively charged, the number of sialic acid groups present in a glycoprotein glycan affects the isoelectric point of the glycoprotein: more the number sialic acids, lower the isoelectric point. Darbepoetin alfa, a novel

glycosylation analog of rHuEPO contains five N-linked carbohydrate chains and up to 22 sialic acids. In contrast, recombinant human EPO has 3 N-linked carbohydrate chains and a maximum of 14 sialic acids (Egrie JC., and Browne JK., Br. J. cancer 2001; 84, 3-10 and Elliot S, et.al., Blood 2000;96:8 2a). As a result of the additional glycosylation (and sialylation), darbepoetin has decreased receptor-binding activity, but exhibits a three-fold longer serum half-life and increased in vivo activity as compared to recombinant human EPO.

rHuEPO produced in Chinese hamster ovary (CHO) cells can exhibit a variable extent of glycosylation and sialylation. (Takeuchi et al., 1989 PNAS 86(20):7819-22, Zanette et al., 2003 Journal of Biotechnology 101(3):275-287). Given that sialylation of erythropoietin is necessary for its *in vivo* bioactivity, consistency in glycosylation and higher levels of sialylation of rHuEPO and its analogs are desirable attributes when producing these recombinant proteins for therapeutic purposes. Thus there is a need for methods that improve glycosylation and sialylation of these glycoproteins in cell cultures.

The literature describes methods for increasing glycosylation of glycoproteins by culturing cells expressing the same in media comprising metal ions. U.S. Patent No. 6,528,286 discloses a process for the preparation of glycoprotein in which the sialic acid content in glycoprotein is increased by addition of copper ions to the cell culture. However, the patent does not describe the use of divalent manganese ions or trivalent iron ions.

U.S. Patent Application Publication No. 2007/0161084 describes a method for producing erythropoietic compositions with some increase in sialylation by culturing cell lines expressing an erythropoietic protein in medium comprising 0.01 to 50 µM concentration of manganese. However the application reports increase in the toxicity for cells at concentrations greater than 60 µM.
WO03045995 relates to a procedure for producing recombinant therapeutic glycoproteins such as human erythropoietin (Epo) using recombinant cell lines.

### SUMMARY

The present invention provides a process for increasing the levels of low pi isoforms of darbepoetin alpha, comprising culturing Chinese hamster ovary cell
lines expressing said darbepoetin alpha, in a medium comprising trivalent iron (Fe³⁺) ions having concentration in the range of 9 µM to 40 µM wherein the darbepoetin alpha so obtained demonstrates an increase in low pl isoforms in comparison to darbepoetin alpha obtained from cells cultured in absence of said concentration of iron (Fe³⁺) as determined by isoelectric focusing.

The culture medium is optionally serum-free and may comprise one or more supplementary amino acids such as asparagine, aspartic acid, cysteine, cystine, isoleucine, leucine, tryptophan, or valine. The host cells may be grown in any suitable cell culture systems such as in roller bottles.

### BRIEF DESCRIPTION OF THE DRAWINGS

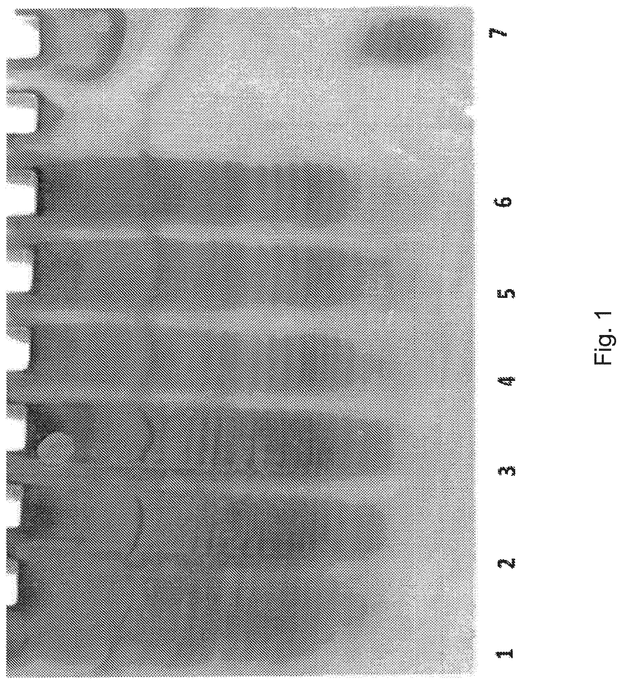

Fig. 1 shows an IEF gel, performed as described in Example 2, of harvest of cells cultured in medium devoid of either manganese ions or trivalent iron ions (Example 1A). Lanes 1-6 are different harvest batches. Lane 7 is a sample of *A*ranesp® used as control. The isoforms of Aranesp® have a pI range of 3-3.9 *(see* Franc oise Lasne et al., Analytical Biochemistry 311 (2002) 119-126*).*

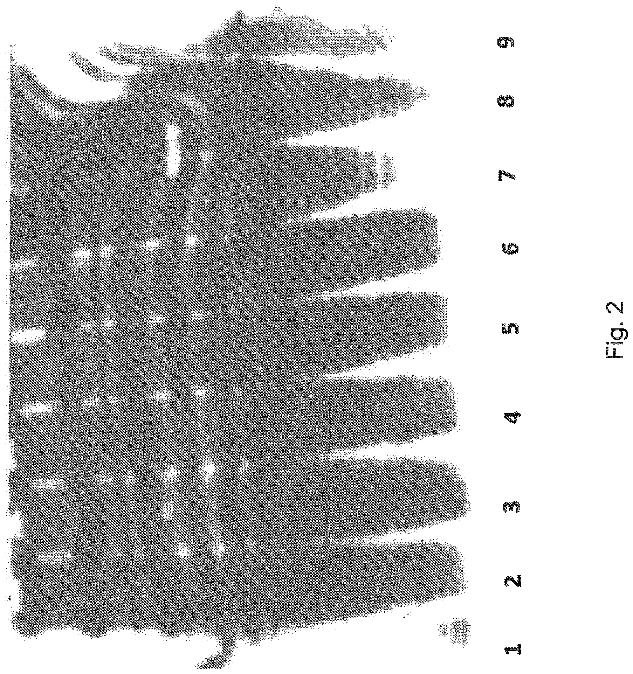

Fig. 2 shows an IEF Gel gel, performed as described in Example 2, of harvest of cells cultured in medium supplemented with 100 µM manganese chloride but devoid of trivalent iron ions. Lane 1 is Aranesp® and lanes 2-9 are the darbepoetin alfa samples showing low pI isoforms expressed in the presence of manganese chloride.

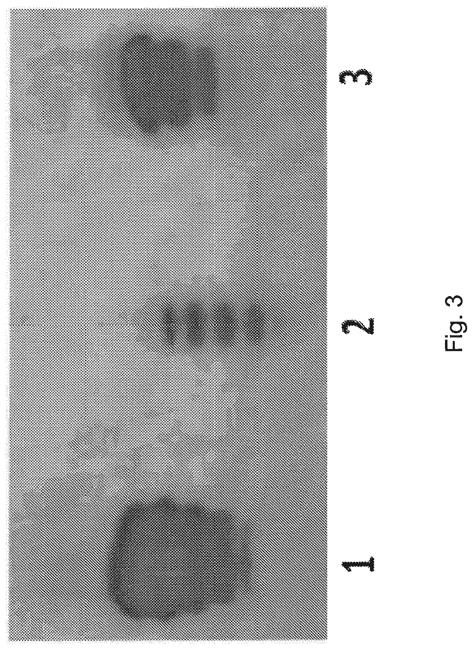

Fig. 3 shows an IEF gel performed as described in Example 2 showing the low pI isoforms of darbepoetin alfa. Lane 1 is harvests of cells grown in the presence of 80 µM manganese and lane 3 is with 38 µM ferric ammonium citrate, subjected to purification by methods disclosed in International Application No. PCT/US2009/048239. Purified samples were then analysed by IEF. Aranesp® (Lane 2) was used as a control.

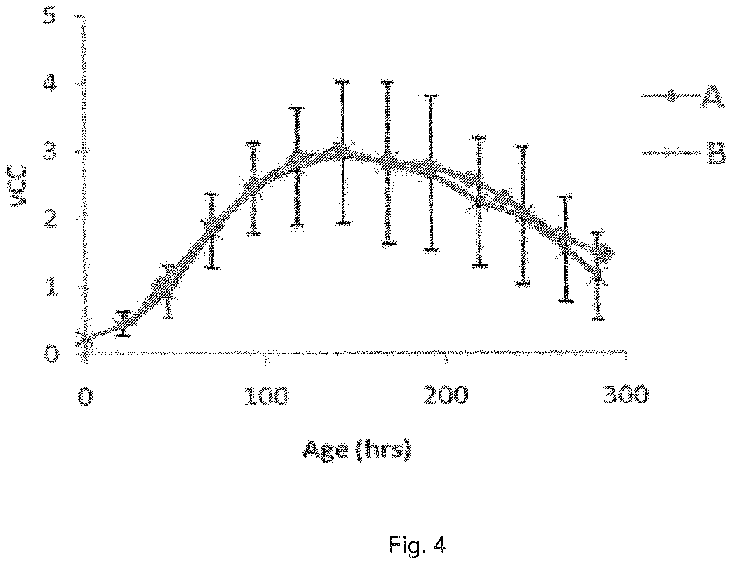

Fig. 4 shows a profile showing the viable cell count and percentage viability against age. Line A represents the VCC and percentage viability of cells cultured in the absence of manganese chloride. Line B represents the VCC and percentage viability of cells cultured in the presence of manganese chloride. Lines A and B show similar viable cell count patterns indicating that higher concentrations of manganese chloride do not affect the viable cell count, and hence is not cytotoxic.

### DETAILED DESCRIPTION

The term "isoform", as used herein, refers to proteins with identical amino acid sequence that differs with respect to charge and therefore isoelectric point, as a result of differences in glycosylation, acylation, deamidation, or sulfation.

The "isoelectric point" or "pi" is the pH at which a particular molecule or surface carries no net electrical charge. The "pI" of a polypeptide refers to the pH at which the polypeptide's positive charge balances its negative charge. The pI can be estimated by various methods known in the art, e.g., from the net charge of the amino acid and/or sialic acid residues on the polypeptide or by using isoelectric focusing, chromatofocusing, etc.

The "low pI isoforms" refer to isoforms with the pI of about 5 or less.

"Non toxic amount or levels" refers to the concentration of metal ions (such as manganese or trivalent ferric ions) in the cell culture media at which there is no reduction in the cell viability, cell growth, or protein production, as compared to media devoid of metal ions.

Erythropoiesis-stimulating proteins, such as human erythropoietin (EPO) and analogs of erythropoietin are glycoprotein hormones that are the principle homeostatic regulators of red blood cell production and are used in the treatment of anemia caused by chronic renal failure.

Darbepoetin alfa, an EPO analog with five N-linked carbohydrate chains and up to 22 sialic acids, exhibits a three-fold longer serum half-life and increased *in vivo* activity as compared to recombinant human EPO. Compositions of erythropoiesis stimulating proteins, such as darbepoetin alfa, comprise a mixture of various isoforms that are known to differ in their extent of glycosylation and sialylation. The low pI isoforms of rHuEPO and darbepoetin alfa, as result of higher sialic acid content, exhibit much higher specific activity as compared to isoforms of higher pI (having low sialic acid content) *(e.g*., N. Imai et al., "Physicochemical and Biological Characterization of Asialoerythropoeitin," European Journal of Biochemistry Vol. 194 (2), pages 457-462, 1990; and EP-A-0 428 267). Hence isoforms with higher sialic acid content and lower pI are of greater therapeutic value. For example, Aranesp® (Amgen Corporation), the approved and marketed form of darbepoietin alpha, comprises essentially low pI isoforms of the protein, in the pI range 3.0-3.9 (see F. Lasne et al., "Detection of isoelectric profiles of erythropoetin in urine:differentiation of natural and administered recombinant hormones," Analytical Biochemistry, Vol. 311 (2), pages 119-126, 2002).

Exemplary sequences, manufacture, purification and use of darbepoetin and other erythropoietin analogs are described in a number of patent publications, including, and International Application Publications WO 91/05867 of Strickland et al., WO 95/05465 of Elliott et al., WO 00/24893 of Egrie et al., and WO 01/81405 of Egrie et al.,.

The cell culture medium comprises high levels of trivalent iron (Fe³⁺) ions, which are effective in increasing the levels of low pI isoforms of erythropoiesis stimulating protein without affecting the viability of the cell culture.

The addition of high concentrations of trivalent iron ions to the culture medium results in an increase in the number and yield of low pI isoforms of erythropoiesis-stimulating molecules, such as human erythropoietin, or its biologically active variants, derivatives (including chemically modified derivatives), or analogs, such as darbepoetin.

The present invention provides a process for increasing the level of low pI isoforms of darbepoetin alfa, comprising culturing Chinese hamster ovary cell lines expressing said darbepoetin alfa, in a medium comprising trivalent iron (Fe³⁺) ions having concentration in a range of 9 µM to 40 µM wherein the darbepoetin alpha so obtained demonstrates an increase in low pI isoforms in comparison to darbepoetin alfa obtained from cells cultured in the absence of said concentration of iron, as determined by isoelectric focusing.

In embodiments, the concentration of Fe⁺³ ions in the culture medium ranges from about 9 µM to about 40 µM, or from about 20 µM to about 40 µM, or is about 38 µM.

Examples of sources of trivalent iron ions include, but are not limited to, ferric ammonium citrate (FAC), ferric ammonium oxalate, ferric ammonium fumarate, ferric ammonium maleate, ferric ammonium succinate, ferric citrate, and ferric nitrate monohydrate.

Salts of trivalent iron ions at high non-toxic concentrations are effective in increasing the levels of low pI isoforms of erythropoiesis stimulating protein and but does not reduce the cell viability, cell growth or protein.

Without wishing to be bound by any particular theory, the use of the selenium, poloxamers, or both in a cell culture medium may prevent adverse effects of high concentrations of Fe⁺³ ions on the cells, thereby maintaining cell viability.

Thus, in embodiments, the cell culture media comprise selenium, or salts of selenium such as sodium selenite.

In embodiments, the cell culture media comprise a poloxamer such as Pluronic® F68.

In embodiments, the cell culture media comprise sodium selenite and a poloxamer such as Pluronic® F 68.

The culture media can also comprise any other nutrient ingredient known in the art, such as carbohydrates, including glucose, essential and non-essential amino acids, lipids and lipid precursors, nucleic acid precursors, vitamins, inorganic salts, trace elements including rare metals, and cell growth factors. A culture medium may be a chemically defined medium or may include serum, plant hydrolysates, or other undefined substances. The culture medium may be entirely serum-free or free of any animal-component. Exemplary media used for culturing the cells include DMEM/F-12 (GIBCO®) media about 15.6 g/L. DMEM medium comprises the following inorganic salts: Calcium Chloride, Cupric sulfate, Potassium Chloride, Magnesium Sulfate or Chloride, Sodium Chloride, Sodium Dihydrogen Phosphate, Sodium Bicarbonate, Zinc sulfate; the following amino acids L-Alanine, L-Arginine, L-Asparagine, L-Aspartic acid, L-Cysteine, L-Glutamic acid, L-Glutamine, Glycine, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine; the following lipids and vitamins: Biotin, D-Calcium-Pantothenate, Choline Chloride, Folic Acid, myo-Inositol, Niacinamide, Nicotinamide, Pyridoxine, Riboflavin, Thiamine, Vitamin B12 (cobalamin), Thymidine, Linoleic Acid, Lipoic Acid; and other components including D-Glucose, Phenol Red, Hypoxanthine, Sodium Pyruvate, Putrescine (1,4-diamino butane), and HEPES (4-(2-hydroxyethyl)-1-piperazineethanesulfonic acid). The culture medium may also include supplementary amino acids, e.g. asparagine, aspartic acid, cysteine, cystine, isoleucine, leucine, tryptophan, and valine. The medium may also comprise lipids and/or lipid precursors such as ethanolamine 5.1 µg/L, Soy peptone 1.0 g/L, Wheat peptone 1 g/L, Fructose 1.0 g/L, sodium bicarbonate 1.2 g/L, Pluronic® F-68 1.0 g/L, HyQ® (HyClone Cell Boost™ 2, Catalogue# SH30890) 35 mL/L, sodium selenite 5 µg/L, and insulin 5 mg/L. In an embodiment, the addition of MnCl₂ is done after a period of rapid growth phase of about 9 days along with the HyQ® feed of about 35 mL/L.

The following examples will further illustrate certain specific aspects and embodiments of the invention in greater detail, and are not intended to limit the scope of the invention.

### EXAMPLE 1

### Preparation of the clone

Darbepoetin alfa producing cell lines were made by transduction of the CHO-S parental cell line with expression retrovector encoding gene of darbepoetin alfa. Following transduction, the pooled population of cells expressed up to 36 µg/ml of darbepoetin alfa after 10-14 days in T-flasks. The pooled population of cells were diluted to very low cell density (1-3 viable cells/200 µL of medium) and plated in 96 well microtiter plates to establish clonal cell lines that originated from single cells. Clones were screened for darbepoetin alfa production and the clones with high productivity were selected for expression.

### Comparative EXAMPLE 1A

### Expression of darbepoetin alfa in the absence of either manganese or trivalent ferric ions

The cells expressing darbepoietin alpha were expanded in spinners and seed reactor before being inoculated into the production reactor. PF CHO HyClone® medium (Catalog # SH30333.03) was used for culturing the cells in the spinner bottle. Table 1 shows the composition of PF CHO medium used for culturing cells. The pH of the medium was 7 and cells from the master cell bank were inoculated at an initial cell count of about 0.2 million cells/mL. The spinner bottles were incubated in a 5% CO₂ and at 37°C for 72 hours. After the spinner stages, cells were inoculated in a 6.5 L seed reactor containing 4 L of SFM-6 medium (composition in Table 2) at an initial cell density of 0.2 million cells/mL, the pH was maintained at 7, temperature at 37.0°C and dissolved oxygen was maintained at 40%. After 72 hours the seed reactor culture was aseptically harvested and the cells were transferred to an 11.5 L production reactor containing 10 L of SFM-6 medium at an initial cell density of 0.2 million cells/mL and fermentation continued under conditions maintained as in the seed reactor described above. The production culture was harvested after 12 days, and supernatant was collected and analyzed.

**Table 1: Composition of (Hyclone®) PF-CHO medium**

| | |
|---|---|
| PF-CHO main powder | 6.0 g, |
| PF-CHO base powder | 10.4 g |
| L-Glutamine | 0.58 g |
| Pluronic® F 68 | 1.0 g |
| Sodium bicarbonate | 2.0 g |
| pH | 7.0 |

| | |
|---|---|
| * PLURONIC F 68 is a copolymer of ethylene oxide and propylene oxide, having an average molecular weight of 8400, and is sold by BASF (Germany). | |

**Table 2: Composition of SFM-6 medium**

| | |
|---|---|
| DMEM/F-12 | 15.6 g |
| MEM amino acids solution (60X) | 10 mL |
| MEM vitamin solution (100X) | 10 mL |
| TE 30 (1000X) | 1 mL |
| Sodium selenite | 5 µg |
| Ethanolamine | 5.1 µg |
| Soy peptone | 1.0 g |
| Wheat peptone | 1.0 g |
| Fructose | 1.0 g |
| Sodium bicarbonate | 1.2 g |
| Pluronic F-68 | 1.0 g |
| HyQ® | 35 mL |
| Insulin | 5.0 mg |

### Comparative EXAMPLE 1B:

### Expression of darbepoetin alfa in the presence of manganese

The culture medium in the seed reactor stage and production stage were supplemented with manganese chloride to a final concentration of 80 to 100 µM. All other conditions were maintained as described in Example 1A. The cell viability so obtained was compared to the viability obtained in Example 1A (see Fig. 3 and Fig. 4).

### EXAMPLE 1C

### Expression of darbepoetin alfa in the presence of trivalent iron ions.

The culture medium in the seed reactor stage and production stage were supplemented with ferric ammonium citrate to a final concentration of 38 µM. All other conditions were maintained as described in Example 1A.

### EXAMPLE 2

### Isoform analysis by IEF

Harvests from Examples 1A, comparative example 1B, and example 1C were analyzed by IEF for the expression of darbepoetin alfa. The gel for IEF was prepared using water, urea, 30% acrylamide, and ampholyte (pH range 2-4 and 3-10). The above components were mixed gently and 10% w/v ammonium per sulphate and TEMED (Tetramethylethylenediamine) were added to mixture and the mixture was casted in gel sandwich apparatus (Bio-Rad Mini-PROTEAN® Cell) and fitted with a comb. The gel was allowed to polymerize for 45 minutes at room temperature. A small amount of this sample was mixed with equal volume of sample buffer (glycerol, ampholyte and Milli Q water) and loaded into the gel. The gel was then placed in Bio-Rad Mini-PROTEAN® Cell assembly and filled with cathode buffer (25 mM sodium hydroxide) and anode buffer (25 mM ortho phosphoric acid) in separate compartments. Samples were run at 200 V for 1.5 hours for pre-focusing of ampholytes at room temperature and then the voltage was increased to 400 V and run for the next 1.5 hours at room temperature. After the run, the gel was carefully removed and stained using either a Coomassie stain or Silver stain. The samples were analyzed (see Fig. 2 and Fig. 3).

## Claims

1. A process for increasing the levels of low pI isoforms of darbepoetin alfa, comprising culturing Chinese hamster ovary cell lines expressing said darbepoetin alfa in a medium comprising trivalent iron ions having a concentration from 9 µM to 40 µM, wherein the darbepoetin alfa so obtained demonstrates an increase in low pI isoforms in comparison to darbepoetin alfa obtained from cells cultured in absence of trivalent iron ions, as determined by isoelectric focusing.

2. A process according to claim 1, comprising culturing cell lines expressing said darbepoetin alfa in a medium comprising non-toxic amounts of trivalent iron ions.

3. A process according to claims 1 to 2, wherein the trivalent iron ions are derived from salts of iron.

4. A process according to any of claims 1 to 2, wherein the trivalent iron ions are derived from ferric ammonium citrate.

5. A process according to any of claims 1 to 4, wherein the medium further comprises selenium or a salt of selenium.

6. A process according to claim 5, wherein the salt of selenium is sodium selenite.

7. A process according to any of claims 1 to 6, wherein the medium further comprises a poloxamer.

## Patentansprüche

1. Verfahren zum Erhöhen des Niveaus von Isoformen von Darbepoetin alfa mit niedrigem pI, das das Kultivieren von Ovarialzelllinien des chinesischen Hamsters, die das genannte Darbepoetin alfa exprimieren, in einem Medium beinhaltet, das dreiwertige Eisenionen mit einer Konzentration von 9 µM bis 40 µM enthält, wobei das so erhaltene Darbepoetin alfa eine Erhöhung der niedrigen pI-Isoformen im Vergleich zu Darbepoetin alfa aufweist, das von Zellen erhalten wird, die ohne dreiwertige Eisenionen kultiviert werden, wie durch isoelektrische Fokussierung bestimmt.

2. Verfahren nach Anspruch 1, das das Kultivieren von Zelllinien, die das genannte Darbepoetin alfa exprimieren, in einem Medium beinhaltet, das nicht toxische Mengen dreiwertiger Eisenionen enthält.

3. Verfahren nach den Ansprüchen 1 bis 2, wobei die dreiwertigen Eisenionen von Eisensalzen stammen.

4. Verfahren nach einem der Ansprüche 1 bis 2, wobei die dreiwertigen Eisenionen von Eisenammoniumcitrat stammen.

5. Verfahren nach einem der Ansprüche 1 bis 4, wobei das Medium ferner Selen oder ein Selensalz enthält.

6. Verfahren nach Anspruch 5, wobei das Selensalz Natriumselenit ist.

7. Verfahren nach einem der Ansprüche 1 bis 6, wobei das Medium ferner ein Poloxamer enthält.

## Revendications

1. Procédé d'augmentation des niveaux d'isoformes à faible pI de darbépoïétine alfa, comprenant cultiver des lignées de cellules ovariennes de hamster chinois exprimant ladite darbépoïétine alfa dans un milieu comprenant des ions de fer trivalent ayant une concentration de 9 µM à 40 µM, dans lequel la darbépoïétine alfa ainsi obtenue fait preuve d'une augmentation en isoformes à faible pI comparée à de la darbépoïétine alfa obtenue de cellules cultivées en l'absence d'ions de fer trivalent, comme déterminé par focalisation isoélectrique.

2. Procédé selon la revendication 1, comprenant cultiver des lignées cellulaires exprimant ladite darbépoïétine alfa dans un milieu comprenant des quantités non toxiques d'ions de fer trivalent.

3. Procédé selon les revendications 1 à 2, dans lequel les ions de fer trivalent sont dérivés de sels de fer.

4. Procédé selon l'une quelconque des revendications 1 à 2, dans lequel les ions de fer trivalent sont dérivés de citrate d'ammonium ferrique.

5. Procédé selon l'une quelconque des revendications 1 à 4, dans lequel le milieu comprend en outre du sélénium ou un sel de sélénium.

6. Procédé selon la revendication 5, dans lequel le sel de sélénium est du sélénite de sodium.

7. Procédé selon l'une quelconque des revendications 1 à 6, dans lequel le milieu comprend en outre un poloxamère.
